# EUROPEAN PATENT APPLICATION

(11) **EP 4 082 599 A1**
(43) Date of publication of application: **02.11.2022**
(21) Application number: 21171636.0
(22) Date of filing: 30.04.2021
(51) Int. Cl.: A61M 39/10, A61M 39/20, A61J 1/14, B65D 41/20, B65D 51/00, A61M 39/00, A61M 39/26, A61M 5/162

(54) **CAP FOR A FLUID CONTAINER, FLUID CONTAINER COMPRISING SUCH CAP AND METHOD FOR MANUFACTURING SUCH CAP**

(71) Applicant: B. Braun Melsungen AG, 34212 Melsungen (DE)
(72) Inventor: VALLOTTON, Raphaël, 1807 Blonay (CH); MILAN, Marco, 1024 Ecublens (CH)
(74) Representative: Prüfer & Partner mbB Patentanwälte · Rechtsanwälte

(57) **Abstract**

Cap (1) for a fluid container (100), comprising: (i) a body (2), (ii) a first port (4), and (iii) a second port (5). The first port (4) comprises a first port housing (41) with a first opening (411) and an elastic first seal member (42) accommodated in the first port housing (41) and sealing the first opening (411). The second port (5) comprises a second port housing (51) with a second opening (511) and an elastic second seal member (52) accommodated in the second port housing (51) and sealing the second opening (511). The first seal member (42) is formed as a pierceable septum (42). The outer surface (421) of the first seal member (42) is flush with an edge surface (412) of the first opening (411). The second seal member (52) comprises a valve opening (522) configured to provide a fluid-tight connection with a fluid manipulation device.

## Description

The present invention relates to a cap for a fluid container, fluid container comprising such cap and method for manufacturing such cap.

For therapeutic purposes, infusions and transfusions are used in human and veterinary medicine. For example, intravenous infusions are used to administer liquids (e.g. solutions of active substances or other liquid pharmaceuticals) into the bloodstream of a patient. For this purpose, the fluid to be administered is taken from a fluid container and flows through an infusion tubing to the intravenous access. The intravenous access can for instance be provided by a peripheral cannula inserted into the patient's median cubital vein. Through the intravenous access, the fluid enters the patient's bloodstream.

An intravenous administration set (also known as "IV administration set", "I.V. administration set", "infusion set", etc.) includes a flexible tube through which the fluid can flow from the container to the intravenous access. An intravenous administration set is a typical example of an administration device, i.e. a device used for administering a therapeutic fluid to a patient. Often, but not necessarily, the intravenous administration set includes a drip chamber connected to the tube so that the fluid flows from the container through the drip chamber into the tube. The intravenous administration set may optionally include further components, for example a flow regulator such as a roller clamp to control the flowrate of the liquid.

If a drip chamber is present, the drip chamber is connected to the container via a container connection so that the liquid can flow from the container into the drip chamber. Otherwise, the tube is directly provided with a container connector.

The container connector can be a piercing device such as a hollow mandrel which can pierce a septum of the container and which has one or more fluid channels in its interior. Such a piercing device is generally referred to as a "piercing spike" or "spike".

A "septum" is a container closure in the form of a membrane or comprising a membrane, wherein the membrane is a rubber membrane or a membrane made of another suitable elastic material. Often, the membrane has the form of a disk inserted into a housing to close its opening. A symmetrical design, e.g. a cylindrical design, is preferred in view of a simple production process because a symmetrical membrane can be inserted in multiple orientations. The membrane is pierced with a hollow piercing device in order to remove fluid from the container or to introduce a fluid into the interior of the container. The piercing device may be a spike as described above, an injection needle, etc. Preferably, the membrane has self-sealing properties. That is when the piercing device is pulled out of the membrane, the opening pierced by the piercing device, i.e. the puncture hole, closes at least partially, preferably completely. The membrane of a septum therefore has no pre-fabricated opening in the form of a hole or slit into which the piercing device is inserted.

A "fluid" is understood to be a flowable material, in particular a liquid or a suspension.

A container access is generally referred to as "port" in the context of medical fluid containers. A port is therefore a container connection site to which an external device for fluid extraction or fluid addition may be connected in order to establish a fluid connection between the interior of the container and the external device. The port to which the intravenous administration set can be connected is called "administration port".

Medical fluid containers are containers which are intended to contain medical fluids and are made of a material suitable for this purpose. Medical fluids include liquid pharmaceuticals for therapy or diagnosis, liquid pharmaceutical components, nutrients, blood for transfusion, etc.

The fluid container from which the fluid to be administered is taken may be a bottle made of a rigid material such as glass or rigid plastic. Besides, plastic bottles with a certain degree of flexibility or plastic bags have become established on the market. Plastic not only has the advantage of having a lower specific weight and being less fragile than glass. If a sufficiently flexible plastic is used, a further advantage is that the container collapses when liquid is removed. This means that the volume of the container continuously adapts to the decreasing volume of the liquid in its interior so that no additional pressure equalization measures in the form of an air inlet channel or an air inlet opening are required. This not only simplifies the design of the administration device but also avoids the risk of contamination of the interior of the container by incoming air.

Some of the containers available on the market containing fluids for intravenous administration allow the addition of other drugs (so-called "drug admixture"). For example, the admixture is carried out by injecting the drug via a further port (so-called "medication port") on the container which also includes a septum. That is, the drug is injected by means of an injection syringe fitted with a hollow needle, wherein the needle is pierced through the septum. Usually, the medication port is located at a position on the container close to the position of the administration port. This may make it easier to manufacture the container but more difficult or even impossible to connect a syringe or other fluid manipulation device to the medication port and, at the same time, a piercing spike or other piercing device to the administration device.

Containers for infusion liquids made of a polyolefin material are available on the market. These containers have a cap on the container head. During infusion, the container points downwards. The cap may have a single septum that may have identified piercing points as described, e.g. in the international standard ISO 15759.The cap may have two ports next to each other. A cap of this type is also called a "twin port cap". The two ports may be of the same design. It is also possible that the cap has two septa that are different from each other, either with respect to their material or their design, as shown, e.g. in US 2009/0054865 A1. Each port comprises a septum which may be pierced by the spike of an intravenous administration set or a needle of an injection syringe. The user may deliberately choose which one of the two parts is used as the administration port and which one as the medication port. It is also possible that one of two functionally identical or functionally equivalent ports is intended as the administration port, whereas the other port is intended as medication port, wherein the different intended use may be indicated by using labels, symbols, different colours, etc. For practical reasons, the caps used for medical containers have a limited diameter, which makes the simultaneous connection of medication and administration device difficult or impossible in the case of the known caps.

The injection of a liquid into the container by means of an injection syringe may be cumbersome because a suitable injection needle has to be kept in stock, unpacked, connected to the injection syringe before use and discarded after use. Further, piercing the needle is prone to incorrect handling. In particular if the piercing direction is not vertical to the septum, the injection needle may be bent and thereby damaged and/or the septum may not seal completely after the injection needle has been withdrawn. In addition, handling an injection needle involves a potential risk of injury. If the drug admixture is carried out during the infusion, when the container is then placed upside-down, the port for injecting the drug to be admixtured may be difficult to access. Moreover, the needle is held only in the area of the puncture through contact with the septum material so that the needle and the syringe can be pulled out or fall out unintentionally.

From DE 10 2007 005 407 A1, a cap for a container for medical liquids is known which has an admixture portion and a withdrawal portion. The withdrawal portion has a pierceable membrane through which a spike of an intravenous administration set can be pierced in the conventional manner. The admixture portion is designed to receive the male cone of a syringe. The admixture portion does not have a septum but a slit valve, i.e. a membrane which has a slit that is opened by the inserted male syringe cone. The cap of DE 10 2007 005 407 A1 and especially the admixture portion (slit valve) and the withdrawal portion (septum) are difficult to clean and disinfect. Hence, germs, endotoxins, disinfectants, etc. may remain and increase the risk of contaminating the liquid prior to its administration to a patient. Moreover, the admixture portion and the withdrawal portion are close to each other and point into the same direction so that simultaneous drug admixture and intravenous administration of the liquid are difficult or impossible. Moreover, the openings of the administration portion and the withdrawal portion are sealed by break-off parts which are complicated to manufacture and cumbersome to handle. Moreover, the liquid is in contact with the septum during storage prior to use, which carries the risk that components of the septum migrate into the liquid. Furthermore, the elastic elements (septum and slit valve) cannot be mounted from the outside.

In view of the situation described above, an objective of the present invention is to provide an improved cap for a medical fluid container, an improved medical fluid container, in particular an improved container for an infusion liquid, and an improved method for manufacturing the cap.

This objective is achieved by the cap according to claim 1, the fluid container according to claim 10, and the method according to claim 14. Refinements of the invention are specified in the dependent claims. Any feature set forth in the dependent claims as well as any feature set forth in the description of exemplary embodiments of the invention below can be understood as a feature suitable for refining the cap, the fluid container, and the method. If possible from a technical point of view, the features of all described embodiments may be combined with one another.

The cap according to the invention is a cap for a fluid container. In particular, the cap is a cap for a container for a medical fluid. The cap comprises: (i) a body, (ii) a first port, and (iii) a second port. The body comprises a container connection portion adapted to be tightly connected to a container opening of a container body. The first port comprises a first port housing. The first port housing comprises a first opening. The first port further comprises an elastic first seal member accommodated in the first port housing and sealing the first opening. The first seal member is formed as a pierceable septum. The outer surface of the first seal member is essentially flush or entirely flush with an edge surface of the first opening. The second port comprises a second port housing. The second port housing comprises a second opening. The second port further comprises an elastic second seal member accommodated in the second port housing and sealing the second opening. The second seal member comprises a valve opening configured to provide a fluid-tight connection with a fluid manipulation device.

Herein, the ordinal numbers "first" and "second" are used to denote those elements that belong to the first port and the second port, respectively, i.e. the "first port housing" is the housing belonging to the first port, etc.

Preferably, the first seal member comprises or is made of an elastomeric material.

Preferably, the second seal member comprises or is made of an elastomeric material.

The cap is adapted to be connected to a container body, i.e. a hollow body with an opening such as for instance a bottle. Whereas a bottle or the like is sometimes referred to as a container, it is referred to as a "container body" here because the expression "container" is used for the capped device in the context of the present invention. Hence, the assembly of the container body and the cap connected thereto is referred to as "container" or "fluid container". For connecting the cap to the container body, the container connection portion of the cap is connected to an opening of the container body which is referred to as its "mouth". The connection between the cap and the container body may for instance be achieved by fixing the connection portion of the cap to a wall portion of the container body defining its mouth such as a neck portion. It is also possible that the connection portion of the cap is fixed to an intermediate part connected to the container body, in particular a moulded transition part. For instance, such transition part may be connected on one of its sides to a flexible bag and on its other side to the cap. The cap might have a substantially oval connection portion that is meant to be welded to a baseport that has a matching receiving distal portion on one side and an almond shaped proximal portion on the other side to be welded on the inner side of plastic films of a bag.

For connecting the cap to the container body, various techniques may be used such as, e.g. welding, gluing, and/or screwing. The mouth allows access to the inside of the container body. Apart from the mouth, the container body may comprise further access sites. The shape of the container connection portion of the cap and the portion at the opening of the container body are complementary, so that there is preferably a fluid-tight connection between the container and the cap.

The first seal member is formed as a pierceable septum. A septum is a closure typically comprising an elastic material such as rubber or other elastomeric material. The septum can be pierced (i.e. penetrated) by a piercing device such as a hollow spike of an infusion set. Such piercing device may also be referred to as "puncture device". The septum seals the first opening, i.e. under normal conditions of use, no fluid can flow through the first opening if the septum has not been pierced by a piercing device. If the septum is pierced by a hollow piercing device, the fluid can flow through a channel within the hollow piercing device, wherein preferably the material of the septum at the piercing site is tight against the outer surface of the piecing device so that no fluid can flow alongside the piercing device but exclusively through the channel of the piercing device. Preferably, the septum is self-sealing, i.e. a channel formed by the piercing device in the material of the septum automatically closes after the piercing device has been withdrawn from the septum, at least if it is pierced by a piercing device up to a certain maximum diameter. This self-sealing function can be achieved for instance by selecting a suitable elastic material. The self-sealing property may also be referred to as "resealing" property.

The outer surface of the first seal member is essentially flush or entirely flush with an edge surface of the first opening.

The outer surface of the first seal member (septum) is the surface which points towards the outside when the cap is connected to a container body, i.e. the surface which does not point towards the inside of the container and which also does not abut the housing. If the cap or the container comprising the cap is oriented so that the first port is at the top, the outer surface may also be referred to as the "top surface" of the first seal member. The outer surface may also be referred to as the "distal surface" of the first seal member.

The expressions "distal" and "proximal" mean closer to and, respectively, further away, from the interior of the container body in an assembled state in which the cap is connected to the container body. In preferred embodiments, the body of the cap has a hat-like structure. The concave side is also referred to as "proximal side" or "inner side" (i.e. the side that points towards the interior of the container if the cap is connected to a container body). The convex side is also referred to as "distal side" or "outer side". In a similar manner, the body's surface on the concave side is also referred to as "proximal surface" or "inner surface" etc.

The edge surface is that portion of the surface of the first port housing which surrounds the inside width of the opening. Therefore, the edge surface defines the border of the opening. If, for instance, the first port housing is formed as a cylindrical or conical protrusion protruding from the body of the cap, the edge surface corresponds to the distal end surface of the first port housing, which may also be referred to as "front surface". The edge surface may have the shape of a circular or elliptical ring or a different circumferential shape.

In preferred embodiments, the first port housing is formed as a hollow protrusion protruding from the remaining body of the cap. The fist seal member is accommodated in the hollow protrusion. The edge surface, viewed in a distal-proximal direction, is the front surface of the protrusion.

The outer surface of the first seal member is entirely flush with the edge surface of the first opening, if these two surfaces merge continuously into each other so that, along the circumference of the first seal member, no step is formed in the area where the surface of the first seal member and the surface of the first port housing meet each other. This means that the outer surface of the first seal member is in alignment with the edge surface of the first opening. The outer surface of the first seal member is essentially flush with the edge surface of the first opening, if a step of 1.5 mm or less, preferably 1.25 mm or less, more preferably 1.0 mm or less, most preferably 0.25 mm or less is formed. In other words: The outer surface of the first seal member is entirely flush with the edge surface of the first opening, if neither surface projects or recedes relative to the other surface. The outer surface of the first seal member is essentially flush with the edge surface of the first opening, if one of the surfaces in question projects or recedes relative to the other surface by a small offset (1.5 mm or less). It is also possible that the surfaces merge continuously into each other but not along the circumference but only along at least 80%, preferably at least 90%, more preferably at least 95% of the circumference of the first seal member. This situation may also be referred to as "essentially flush".

Due to the alignment of the outer surface of the first seal member and the edge surface of the first opening, which are entirely flush or essentially flush with one another, the process of disinfection of the first seal member is made easier and safer. Because no significant discontinuous surface structure such as a step is present in the area to be disinfected, an effective disinfection by applying a disinfectant by rinsing, spraying, wiping, or other application technique can be achieved. The disinfectant can efficiently reach the entire area to be disinfected. This prevents pathogens and other contaminants from accumulating in a surface discontinuity and thus prevents the accumulation of contaminants that cannot be removed effectively or cannot be removed at all by the application of a disinfectant. In addition, a cavity is avoided in which disinfectant could accumulate after application. Such an accumulation of disinfectant ("disinfectant pool") is unfavourable when using the cap or a container with such cap because either it is necessary to wait for the evaporation of the disinfectant after disinfection and before piercing through the septum with the piercing device, or there is a risk that disinfectant or other debris (various particulate matter, pathogens, dirt, dead organisms, that cannot evaporate, etc.) is carried through the septum together with the piercing device and could thus enter the patient's bloodstream. Moreover, the disinfection carried out in the course of the production of the cap or the container may be easier or more efficient due to this alignment of the outer surface of the first seal member and the edge surface. In addition, a quick disinfectant evaporation may be beneficial in view of production time and it may avoid that disinfectants are trapped between the septum and a sealing foil applied thereon after disinfection. Disinfectants usually are organic solvents such as ethanol or isopropanol. Such organic solvents could damage the septum in particular if trapped between the septum and the foil. A quicker and more efficient radiation-based disinfection may also be achieved due to an entirely or essentially flush alignment because shadow areas are avoided or minimised.

With regard to disinfection efficiency, it is preferred that the outer surface of the first seal member and the edge surface of the first opening are arranged in an entirely flush manner. However, an only essentially flush alignment, for instance with a small offset of 1.0 mm to 1.5 mm, is sufficient, or even might be preferred with regard to the robustness of the process of assembling the septum and the first port housing. Such small offset, if present, is tolerable with regard to disinfection efficiency.

Due to the provision of both ports on the same base part (body), an efficient fabrication of fluid containers is possible because the container body has to be connected to only one part. In addition, the body may be adapted to different container mouth shapes.

The second seal member comprises a valve opening configured to provide a fluid-tight connection with a fluid manipulation device. A fluid manipulation device is a device with which fluid such as a liquid can be removed from the container or with which fluid can be added to the container. The fluid manipulation device may be, for example, a syringe without a needle attached. The valve opening is configured to be connected to the male connector of the fluid manipulation device. Fluid transfer is then possible between the interior of the container body connected to the cap and the fluid manipulation device. Preferably, the connection between the male connector of the fluid manipulation device and the valve opening is fluid-tight, i.e. fluid can flow through the valve exclusively via the male connector of the fluid manipulation device. In other words, the connection between the fluid manipulation device and the valve opening is fluid-tight if it does not leak, i.e. the material around the valve opening is tight against the outer surface of the male connector of the fluid manipulation device.

The configuration of the valve opening depends on how the male connector of the device with which a connection is intended is formed. For example, the device may be a needleless syringe provided with a male connector in form of a cone or another male mating member. If the connection with such a syringe is to be possible, the valve opening may be, for example, a slit into which the male mating member is inserted to make the connection. The cone of a syringe may correspond to the standardised Luer geometry or deviate from the Luer geometry. Preferably, the valve opening is designed in such a way that a fluid-tight connection with differently designed male connectors is possible, e.g. with male Luer cones or different male mating structures.

According to embodiments of the invention, the valve opening is formed as a slit intended to be penetrated by a male connector. According to other embodiments, the second seal member is configured such that the male connector does not penetrate through the slit but deforms the upper surface of the second seal member in a way the slit opens.

Preferably, the valve opening is resealable, i.e. after disconnecting the fluid manipulation device, the valve opening automatically closes such that the second elastic member seals the second opening in a fluid-tight manner again.

In preferred embodiments, the first port is an administration port, i.e. a port for withdrawing a liquid that is to be administered to a patient from the container body connected to the cap. For instance, the first seal member is adapted to be pierced by a spike of an administration device such as an infusion set, a transfusion set, a transfer device etc. By piercing the first seal member by the spike, a fluid connection between the interior of the container body connected to the cap and the administration device is established.

In addition or alternatively, in further preferred embodiments, the second port is a needle-free port, i.e. a port through which a fluid connection with a syringe that is not provided with a needle or a similar device can be made. In other words, the second port is in this case not intended to be pierced by a needle or another device in order to provide a fluid connection via the second port.

It is preferred that the fluid connection between the interior of the container body connected to the cap and the syringe or the other fluid manipulation device is a bidirectional fluid connection, i.e. fluid can flow through the valve opening either into the container body or out of the container body.

In addition or alternatively, in further preferred embodiments, the first seal member and/or the second seal member is overmoulded onto the body of the cap. Overmoulding is a method in which a material is added to an already existing object by moulding in order to produce an assembled product. The already existing object may be an object produced by injection moulding or any other production technique.

In addition or alternatively, in further preferred embodiments, the outer surface (distal surface) of the first seal member is essentially flat or entirely flat. Herein, the outer surface is considered to be essentially flat if it has protrusions and/or recesses whose height is less than 10% of the largest diameter of the outer surface. For instance, it is possible that the outer surface has such a recess in order to indicate the location where the user should pierce the first seal member.

Preferably, the diameter of the first seal member (measured transversely to the direction in which it is pierced) is large enough such that the elastic material in the space between a piercing device such as a spike pierced through the first seal member and the first port housing does not need to be squeezed upon insertion of the piercing device thus preventing high friction to the piercing device being inserted and allowing for easy insertion of the piercing device.

Preferably, the diameter of the first seal member (measured transversely to the direction in which it is pierced) is small enough such that a sufficient force for holding the piercing device is ensured.

Preferably, the diameter of the first seal member (measured transversely to the direction in which it is pierced) is smaller than the diameter of the first port housing such that a radial compression of the elastic material results in order to improve resealability after disconnection of the piercing device.

In addition or alternatively, in further preferred embodiments, the second seal member has a diameter measured transversely to the direction in which it is pierced of at least 8 mm, preferably at least 9.5 mm and/or 18 mm or less, preferably 13 mm or less.

The first seal member may also have a non-circular shape. It may, e.g., be elliptical. The above-mentioned preferred diameters are then understood as the smaller dimension of the non-circular shape, e.g. the shorter axis of an ellipse.

Preferably, the thickness of the first seal member (measured in the direction in which it is pierced) is chosen to ensure sufficient resealability and sufficient force for holding the piercing device, and to avoid excessive force for the insertion of the piercing device.

In addition or alternatively, in further preferred embodiments, the thickness of the first seal member measured in the direction in which it is pierced is 1.5 mm or more, preferably 2 mm or more and/or 5 mm or less, preferably 4 mm or less.

Preferably, the material of the first seal member is chosen to ensure sufficient resealability and sufficient force for holding the piercing device, and to avoid excessive force for the insertion of the piercing device.

In addition or alternatively, in further preferred embodiments, the first seal member comprises
- rubber, preferably vulcanized rubber, and/or
- at least one thermoplastic elastomer, more preferably at least one thermoplastic elastomer selected from the group consisting of styrenic block copolymers, thermoplastic polyolefin elastomers, thermoplastic vulcanizates, thermoplastic polyurethanes, thermoplastic copolyester, and thermoplastic polyamides.

In addition or alternatively, in further preferred embodiments, the first seal member comprises an elastic material having a hardness between 25 and 55 shore A.

In addition or alternatively, in further preferred embodiments, the first seal member comprises an elastic material including pigments and/or dyes capable of absorbing electromagnetic radiation. The presence of substances having the ability of absorbing electromagnetic radiation may for instance improve the usability of laser welding for connecting the first seal member to the first port housing.

In addition or alternatively, in further preferred embodiments, the diameter of the second seal member (measured transversely to the direction in which the fluid manipulation device is connected to the second port) is 18 mm or less, preferably 13.5 mm or less.

The second seal member may also have a non-circular shape. It may, e.g., be elliptical. The above-mentioned preferred diameters are then understood as the smaller dimension of the non-circular shape, e.g. the shorter axis of an ellipse.

In addition or alternatively, in further preferred embodiments, the length of the second seal member (measured in the direction in which the fluid manipulation device is connected to the second port) is between 10 and 15 mm and/or longer than the cavity in which it is accommodated to create an axial pre-load to ensure (1) sufficient elastic force such that the second seal member returns to its initial position when the fluid manipulation device is disconnected and (2) sufficient sealing. Herein, the force that the user has to exert onto the fluid manipulation device in order to connect the same to the second port should be moderate for the sake of good usability.

In addition or alternatively, in further preferred embodiments, the second seal member comprises a distal wall in which the valve opening is formed and a blind hole open at the proximal portion of the second seal member and terminating at the distal wall such that the interior of the blind hole is in communication with the valve opening.

Preferably, the thickness of the distal wall is 1 mm or more and 6 mm or less. A thinner wall would possibly be less suitable in view of resealability and tightness, especially under pressure conditions. A thicker wall could be more complicated for production and cost more. More preferably, the wall thickness is between 1 mm and 3 mm.

Preferably the diameter of the blind hole is 2 mm or more to ensure proper fluid flow. More preferably, the diameter of the blind hole is 2.5 mm or more.

Preferably, the material of the second seal member is selected to ensure (1) easy connection with the fluid manipulation device, (2) sufficient force such that the second seal member returns to its initial position when the male connector is disconnected, (3) resilience, i.e. minimum change of material properties, even after repeated connection of a fluid manipulation device, (4) high tear resistance to prevent tearing upon insertion of the male connector.

In preferred embodiments the second seal member comprises
- rubber, preferably vulcanized rubber, more preferably vulcanized polyisoprene rubber, and/or
- silicone, preferably silicone rubber, and/or
- at least one thermoplastic polymer, preferably at least one polymer selected from the group consisting of styrenic block copolymers, thermoplastic polyolefin-elastomers, thermoplastic vulcanizates, thermoplastic polyurethanes, thermoplastic copolyester, and thermoplastic polyamides.

In addition or alternatively, in further preferred embodiments, the second seal member comprises an elastic material having a hardness between 25 and 55 shore A.

In addition or alternatively, in further preferred embodiments, the body comprises a polyolefin, preferably polypropylene and/or polyethylene.

In addition or alternatively, in further preferred embodiments, the body comprises a polymer material having a tensile modulus and/or flexural modulus of at least 700 MPa.

In addition or alternatively, in further preferred embodiments, the second seal member provides a fluid-tight connection with the fluid manipulation device, in particular a releasable connection such that the fluid-tight connection and disconnection of a fluid manipulation device is possible multiple times.

In addition or alternatively, in further preferred embodiments, the valve opening is self-sealing, i.e. the valve automatically closes after the male connector (e.g. syringe cone) of the fluid manipulation device has been disconnected. Such self-sealing valve opening may also be referred to as "resealing valve opening". Preferably, the self-sealing property is provided or improved by preloading the second seal member by an elastic deformation providing a spring force causing the valve opening to close after the male connector has been withdrawn from the valve opening. The preloading may have an axial force component, i.e. a force component in the direction in which the male connector of the fluid manipulation device is inserted. The axial force component may be provided by choosing the axial dimensions of the second port housing and the second seal member such that the former is shorter than the latter resulting in an axial compression. Alternatively or additionally, the preloading may have a radial force component, i.e. a force component perpendicular to the axial direction. The radial force component may be provided by a conical structure of the inner surface of the second port housing and/or by a lumen of the inner surface of the second port housing that is smaller than the dimensions of the outer surface of the second seal member.

In addition or alternatively, in further preferred embodiments, the second port is formed as female mating connector, i.e. it is adapted to be connected to a male mating connector used as connection part of the fluid manipulation device. The male mating connector may, for instance, be a connector designed according to any sub-part of the ISO 80369 series of international standards, in particular a male Luer connector according to ISO 80369-7, a male connector according to ISO 80369-6, or a similar design. The connection system according to ISO 80369-6 is also referred to as NRFit^{®}. Preferably, the female mating connector is formed in a way to allow tight connection with such a male connector. Connector geometries different from the Luer geometry are, however, also possible.

The second port housing may comprise a thread structure, preferably a thread structure adapted for connecting with a male Luer lock structure.

In addition or alternatively, in further preferred embodiments, at least a portion of the second port housing is formed as a separate housing element connected to the body of the cap, i.e. the separate housing element is not formed monolithically with the body of the cap. The second seal member is at least partially accommodated in the separate housing element. The separate housing element is connected to the body, preferably in a permanent manner. In this context, a permanent connection is a connection that can only be disconnected in a destructive manner. For the connection of the separate housing element, a connection socket formed monolithically with the body of the cap may be provided. More preferably, the separate housing element is permanently connected to the body (in particular to the socket, if present) by welding, e.g. to the connection socket.

Preferably, the cap further comprises a membrane. Preferably, the membrane is formed of the same material as the body of the cap, in particular monolithically with the body of the cap. The membrane is arranged underneath the first seal member seen in a direction of insertion of a piercing device, wherein the membrane is adapted to be pierced by the same piercing device that penetrates the first seal member. That is, when the piercing device is connected to the fluid container, it penetrates the first seal member and, subsequently, the membrane. Such membrane may have the ability to prevent contact between the liquid and the first seal member in order to prevent that components of the first seal member are leached and migrate into the liquid prior to use.

The properties of the membrane such as its material and its thickness are selected such that the tightness is on the one hand sufficient during transportation, storage, and manipulation prior to use and on the other hand not too high in order to avoid excessive force required for connecting the piercing device to the container.

In more preferred embodiments, the membrane has a thickness of 0.1 mm or more, particularly 0.15 mm or more and/or 0.5 mm or less, particularly 0.25 mm or less.

Preferably, the cap has a height between 12 mm and 50 mm, more preferably between 14 mm and 30 mm, measured in a direction transverse to the mouth of the container body 200 with which the cap 1 is to be connected. Such height may be seen as a compromise between a user-friendly handling and a low residual volume within the cap 1 and space requirements during transport and storage.

According to preferred embodiments of the invention, the first seal member is accommodated in a seat structure at the distal end of the first port housing and held in place by means of a fastener attached to the distal end of the first port housing.

In the course of the production of a cap of one of the aforementioned embodiments having a separate housing element for the second port, it is possible that the second seal member is mounted by approaching the second seal member from the distal side and putting the separate element over the second seal member. The direction "from the distal side" is the direction corresponding to the direction in which the male connector of the fluid manipulation device is inserted into the second port. Mounting from the distal side may be less complicated than from the opposite side, in particular if the cap has a concave shape so that the mounting tools can reach the distal side more easily. In addition, only by mounting from the distal side, it is possible to mount the second seal member after the cap has already been connected to the container body which may improve the flexibility of the production process.

The fluid container according to the invention, which in particular is a fluid container for a medical fluid, comprises
- a hollow container body having a container body opening portion
- a cap according to the invention.

The body of the cap is in fluid-tight connection with the opening portion.

The fluid container may comprise a cap of any one of the embodiments of the invention.

The method according to the invention is a method of manufacturing a cap according to the invention. The method comprises the steps
A) manufacturing or providing the body,
B) manufacturing or providing the first seal member,
C) manufacturing or providing the second seal member,
D) mounting the first seal member,
E) mounting the second seal member, wherein the outer surface of the first seal member is essentially flush or entirely flush with the edge surface of the first opening,
F) optionally arranging a peel-off foil atop the first seal member and/or atop the second seal member.

Herein, the first seal member may be mounted after or before the second seal member. It is also possible that both seal members are mounted at least partially simultaneously in order to save production time.

Mounting of a seal member includes inserting into the respective port housing and, preferably, fixing it in place. For instance, laser welding can be used to fix the first seal member in place. For instance, a form-fit connection by the second port housing may be used to fix the second seal member.

Alternatively, the first seal member and/or the second seal member may manufactured in an in-situ manner (i.e. simultaneously manufactured and mounted) by overmoulding it in the respective portion of the body.

The peel-off foil is removed before a piercing device is pierced through the first seal member. The peel-off foil seals and protects the respective sealing member, and in particular provides tamper evidence because the peel-off foil makes it easy to determine whether the sealing member is damaged or has already been connected to a fluid manipulation device or an administration device, respectively. Furthermore, the peel-off foil provides additional tightness for the respective port and helps to ensure its mechanical integrity, for example, when the cap is subjected to mechanical stress. Compared to a break-off part, a peel-off foil is less subject to damage. This is due to its flat construction. Further, less material is needed for a peel-off foil. Further, the use of a peel-off foil leads to a shorter container, which is advantageous for storage and transportation.

For inserting the first seal member, preferably the first seal member is inserted into the first port housing from the distal side, i.e. in a direction corresponding to a direction in which a piercing device is inserted into the first port and fixed in place, e.g. by laser welding. In alternative embodiments, the first seal member may be inserted into the first port housing in the opposite direction, i.e. from the interior of the body.

For mounting the second seal member, preferably the second seal member is brought into position from the distal side, i.e. in a direction corresponding to a direction in which the male connector of a fluid manipulation device is inserted into the second port, wherein preferably the second port housing is attached atop the second seal member in order to fix the second seal member in place. In alternative embodiments, the second seal member may be inserted into the first port housing in the opposite direction, i.e. from the interior of the body.

In step A, the body may be provided or manufactured as a body connected to a container body, the latter optionally filled with a liquid, before the first seal member, the second seal member, or both seal members are mounted. In this case, it is especially beneficial or even necessary that the seal member(s) yet to be mounted can be inserted into the respective port housing from a distal side, because the port housings are not accessible for inserting the seal members from within the container. This in turn means that the cap according to the invention offers the advantage of being able to be processed in the course of manufacturing a container in such a way that the seal members can be mounted from the outside (from a distal side), which brings advantages in terms of the flexibility of the manufacturing process.

Other features and expediencies of the invention may be found in the description of exemplary embodiments with the aid of the appended drawings.
- Fig. 1: shows a schematic sectional view of a cap according to an embodiment of the invention.
- Fig. 2: shows a schematic perspective view of the cap according to this embodiment.
- Fig. 3: shows the same sectional view of the cap according to this embodiment as Fig. 1, wherein the ports of the cap are provided with peel-off foils.
- Fig. 4: shows a schematic sectional view of a container according to a further embodiment of the invention, wherein the container comprises a cap according to the embodiment shown in Figures 1 and 2.
- Fig. 5 (a) and Fig. 5 (b): show a schematic sectional view of a lateral distal portion of the first port according to different embodiments of the invention.
- Fig. 6 (a) and Fig. 6 (b): show a schematic perspective view and a schematic sectional view, respectively, of a cap according to a further embodiment.
- Fig. 7 (a) and Fig. 7 (b): show a schematic perspective view and a schematic sectional view, respectively, of a cap according to a further embodiment.

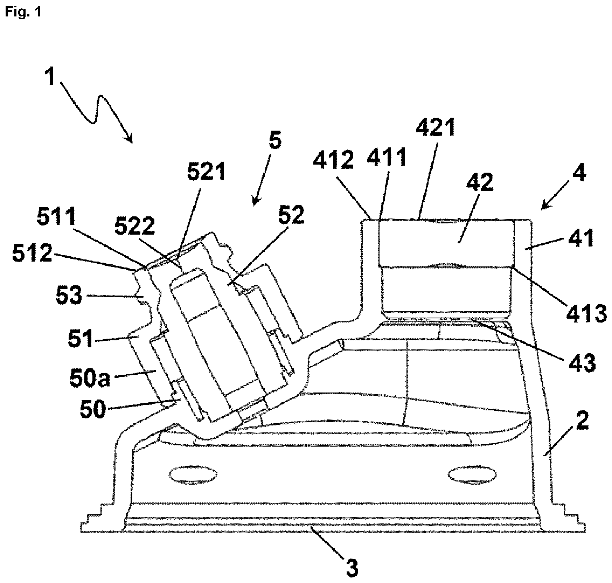

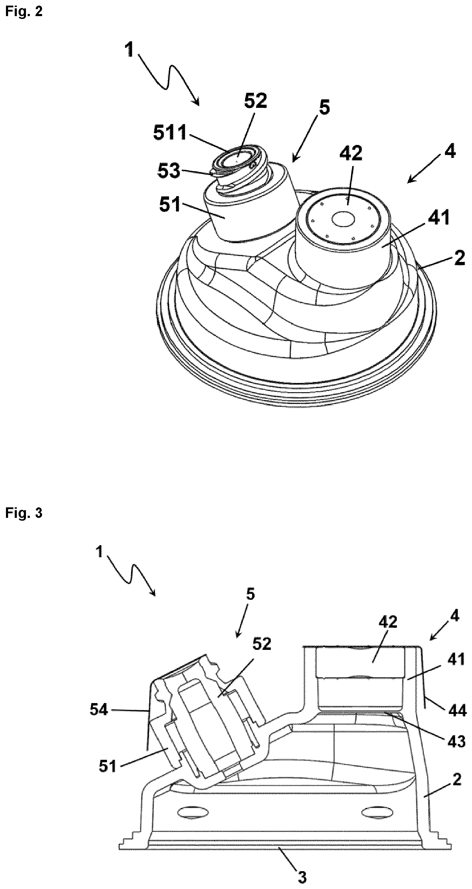

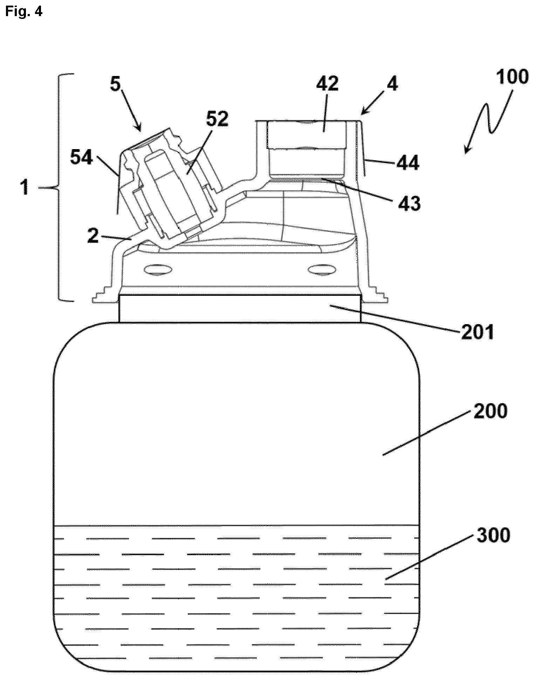

Figures 1 and 2 are a schematic views of a cap 1 according to a first embodiment of the invention. The cap 1 is intended to be attached to the opening 201 of a container body 200. Together, the cap 1 and the container body 200, onto which the cap is mounted as a container closure, form a container 100 according to the invention. The container 100 including a cap 1 according to the first embodiment is shown in Fig. 4. The container 100 shown in Fig. 4 contains a liquid 300 that is not part of the container 100. The opening 201 of the container body 200 is formed as a container neck, of which an axial portion is enclosed by a connection portion 3 of the cap 1. Other configurations of the container body's opening structure and the connection portion are possible, e.g. a neck that is entirely enclosed by the connection portion 3 or a neck-less opening to a front surface of the cap 1 serving as connection portion 3 is attached by glue, welding, etc.

In Fig. 4, the container body 200 is shown as a bottle. In other embodiments, the container body may be a fluid reservoir of another type such as, e.g., a more or less flexible bag with an optional intermediate part forming the mouth of the bag to which the connection portion 3 is fixed.

Preferably, the fluid 300 is a medical fluid such as a liquid drug. In other words, it is preferred that the container 100 is suitable to contain a medical fluid and suitable to be used in a medical environment.

Preferably, the container 100 is tightly closed if both parts are in their closed state, i.e. if neither an administration device nor a fluid manipulation device is connected to the respective port. In use, e.g. during administering an intravenous infusion, the container 100 is usually in an upside-down orientation, that is, the cap 1 is then located at the lowermost position so that the liquid 300 can flow from the container body 200 into the administration device connected to the first port 4. For adding a fluid to the inside of the container 100 through the second port 5 using a fluid manipulation device, in general no specific orientation of the container 100 is required.

The cap 1 comprises a body 2 constituting the main part of the cap 1. In the illustrated embodiment, the body 2 is shown as a hollow cup-like structure with
- an opening at the connection portion 3 on the bottom side in the orientation shown and
- protrusions positioned on an elevated section on the body's upper side in the portions where the ports 4, 5 (see below) are formed.

The elevated section and the protrusions are optional.

In the illustrated embodiment, the lower part of the cap 1 is circular because the cap 1 according to this embodiment is intended to be attached to a container body 200 with a circular opening 201. Other shapes of the lower part of the cap 1 are possible for other shapes of the opening, e.g. elliptical or rectangular with rounded corners.

In the illustrated embodiment, the connection portion 3 is shown to be a tapered ring-like portion of the inner surface of the cap 1. In other embodiments, the connection portion may include, for instance, a cylindrical inner surface portion, an inner surface portion with a step, a front surface at the lower edge of the cap 1, etc. It is also possible that the connection portion 3 is deformed when the cap 1 is connected to the container body 200, in particular if welding or another heat-involving process is used therefor.

The cap comprises two ports 4, 5. The first port 4 may, e.g., serve as administration port. The second port 5 may, e.g., serve as medication port.

Both ports comprise a port housing, wherein both port housings comprise a respective opening: The first port 4 comprises a first port housing 41 with a first opening 411 and the second port comprises a second port housing 51 with a second opening 511.

The openings 411, 511 provide access points for fluid transfer into or out of the container 100, if the cap 1 is connected to a container body 200. A fluid could flow through the first opening 411 or the second opening 511, if the first seal member and the second seal member, respectively, were removed from the port housings 41, 51. The seal members accommodated in the port housings 41, 51 ensure that fluid may only be transferred through the openings by using a piercing device and/or a fluid manipulation device. The seal members, the piercing device, and the fluid manipulation device will be described further below.

The piercing device and the fluid manipulation device are not part of the container according to the present invention.

In the embodiment shown in Figures 1 and 2, the first port housing 41 is a hollow circular cylindrical protrusion formed monolithically with the body 2, wherein the upper edge of the first port housing 41, i.e. the circular front surface 412, defines an opening 411.

In further embodiments, the cylindrical protrusion has a non-circular cross section, e.g. an elliptical cross section. In further embodiments, the protrusion is conical.

In further embodiments, the fist port housing is not formed monolithically; instead, at least a portion of the first port housing is formed as a element that is attached to the remaining body of the cap, for instance by laser welding.

In the first port housing 41, an elastic first seal member 42 is accommodated. In the embodiment shown in Figures 1 and 2, the distal portion of the first port housing 41 is formed slightly broader, so that an edged or rounded step 413 is formed on the inner wall of the first port housing 41. The septum 42 is fitted into this broader section and abuts the step 413 with the edge of its proximal side. In alternative embodiments, such a step may be replaced by protrusions or other suitable structures, or may be completely absent.

The first seal member 42 is formed as a pierceable septum 42 that may be pierced by a spike of an infusion set or another piercing device. That is, the septum 42 closes the first opening 411, if no piercing device has been pierced through the septum. If a hollow piercing device is pierced through the septum, a fluid can flow through the piercing device. In other words, the piercing device pierced through the septum acts as fluid entrance or fluid exit of a container 100 if the cap 1 is connected to a container body 200.

Preferably, the septum 42 is self-sealing, i.e. a channel formed by the piercing device in the material of the septum 42 automatically closes after the piercing device has been withdrawn from the septum 42.

The septum 42 before its insertion into the first port housing 41 is slightly larger than the inner dimensions of the port housing 41 and in particular slightly larger than the first opening 411 such that the septum 41 is compressed when accommodated in the first port housing 41 providing an elastic deformation. Such elastic deformation creates as force that improves the self-sealing properties of the septum 42. In addition, the elastic force may be used to hold the septum 42 in the first port housing 41. Alternatively or additionally, the septum 42 may be attached to the first port housing 41 by means of a suitable joining technique, e.g. welding (in particular laser welding), gluing, etc. In addition it is possible to form the septum 42 in situ, e.g. using a curable compound, wherein the compound adheres to the inner wall of the first port housing 41 in the course of curing.

In addition or alternatively, in further preferred embodiments, the septum 42 comprises a material including pigments and/or dyes capable of absorbing electromagnetic radiation in order to improve the usability of laser welding for connecting the septum to the first port housing 41.

Preferably, the size of the septum 42 measured transversely to the direction in which it is pierced is large enough such that the elastic material in the space between a piercing device such as a spike pierced through the septum 42 and the first port housing 41 does not need to be squeezed to much upon insertion of the piercing device thus preventing high friction to the piercing device being inserted and allowing for easy insertion of the piercing device. Further preferably, the size of the septum 42 measured transversely to the direction in which it is pierced is small enough such that a sufficient force for holding the piercing device is ensured.

In addition or alternatively, in further preferred embodiments, the septum has a diameter measured transversely to the direction in which it is pierced of at least 8 mm, preferably at least 9.5 mm and/or 18 mm or less, preferably 13 mm or less.

Preferably, the thickness of the septum 42 measured in the direction in which it is pierced is chosen to ensure sufficient resealability and sufficient force for holding the piercing device, and to avoid excessive force for the insertion of the piercing device. In addition or alternatively, in further preferred embodiments, the thickness of septum 42 measured in the direction in which it is pierced is 1.5 mm or more, preferably 2 mm or more and/or 5 mm or less, preferably 4 mm or less.

In specific embodiments, the first seal member 42 comprises
- rubber, preferably vulcanized rubber, and/or
- at least one thermoplastic elastomer, more preferably at least one thermoplastic elastomer selected from the group consisting of styrenic block copolymers, thermoplastic polyolefin elastomers, thermoplastic vulcanizates, thermoplastic polyurethanes, thermoplastic copolyester, and thermoplastic polyamides.

In addition or alternatively, in further preferred embodiments, the first seal member 42 comprises an elastic material having a hardness between 25 and 55 shore A.

In the embodiment shown in Figures 1 and 2, the outer surface 421 (distal surface) of the first seal member 42 is essentially flat having only minor protrusions and/or recesses. The optional small recess in the centre portion of the surface 421 shown in Figures 1 and 2 indicates the location where the user should pierce the first seal member.

In the embodiment shown in Figures 1 and 2, the first opening 411 is defined by the front surface of the first port housing. That is, the front surface is the edge surface 412 of the first opening 411. In the embodiment shown in Figures 1 and 2, the edge surface 412 has a circular ring-like shape. In alternative embodiments, the edge surface may have a different shape, e.g. an elliptical ring-like shape. In the embodiment shown in Figures 1 and 2, the edge surface is flat and perpendicular to the distal-proximal direction, i.e. the direction in which the piercing device is to be pierced through the septum 42. In alternative embodiments, the edge surface 412 may have a different shape and/or a different orientation. For instance, it may be rounded and/or tapered (i.e. inclined with respect to the distal-proximal direction).

The outer surface 421 (distal surface) of the septum 42 is flush with the edge surface 412 of the first opening 411.

That is, the edge surface 412 and the distal surface 421 of the septum 42 merge continuously into each other so that, along the circumference of the septum 42, no step is formed in the area where the first seal member and the first port housing meet each other.

If both the edge surface 412 and the distal surface 421 of the septum 42 are flat, the flush surfaces lie in the same plane. If, in alternative embodiments, the edge surface is tapered and/or rounded, the flush surfaces merge continuously into each other but do not lie in the same plane.

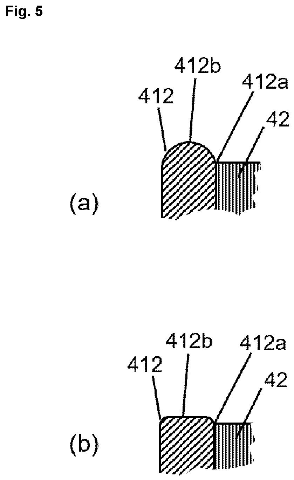

If the edge surface 412 is rounded (as shown in Fig. 5 (a)) or has rounded borders (as shown in Fig. 5 (b)), or has chamfered borders, it is preferred that the distal surface 421 of the septum 42 is not as the same level as the ridge line or ridge surface 412b, i.e. the uppermost line or surface in Figures 5 (a) and 5 (b) but that the rounded portions 412a of the edge surface 412 and the distal surface 421 of the septum merge continuously into each other. The continuous merging at the portions 412 ensures that no groove is formed that may act as a trap for contaminations. The configurations shown in Figures 5 (a) and 5 (b) therefore represent alignments of the edge surface 412 and the distal surface 421 that are preferably considered as flush alignments in the case of a rounded edge surface 412 or an edge surface 412 with rounded borders 412a, and analogously for edge surfaces 412 with chamfered borders or other types of broken borders.

In alternative embodiments, the septum 42 is not entirely flush with the edge surface 412, but only essentially flush in the sense that only a step of 1.5 mm or less, preferably 1.25 mm or less, more preferably 1.00 mm or less, most preferably 0.25 mm or less is formed between the septum 42 and the edge surface 412.

In the embodiment shown in Figures 1 and 2, the second port housing 51 is formed by a bushing 50a connected to a connection socket 50 formed monolithically with the body 2. Preferably, the bushing 50a is permanently connected to the connection socket 50, e.g. by means of an adhesive, welding, etc. The use of ultrasonic welding is preferred, in particular using a shear joint design. This may improve a circumferential welding that is able to resist a rotational torque and a lateral bending force acting on the second port housing 52. For the connection of the bushing 50a to the socket 50, also a form-fit connection (such a snap connection), a screw connection, etc., or a combination of different connection techniques (e.g. form-fit plus adhesive) is possible.

Specifically, the connection socket 50 is formed as a rim that projects beyond the surface of the body 2 in both directions. The connection socket 50 provides a proximal stop forming a retaining structure preventing the second seal member 52 from being pushed further into the interior of the body 2. The connection socket 50 provides a connection portion for the connection with the bushing 50a. In alternative specific embodiments, the connection socket 50 does not project beyond the surface of the body 2 either on its distal side or its proximal side.

In alternative embodiments, no connection socket 50 is provided such that the bushing 50a is directly connected to the outer surface of the body 2.

In further alternative embodiments, the second port housing comprises a protrusion formed monolithically with the body 2 and protruding from the outer surface of the body 2. A distal element is connected to the protrusion so that the protrusion and the distal element together form the second port housing. The protrusion may be cylindrical with a circular or a non-circular (e.g. elliptical) cross section, conical, etc. The distal element holds the second seal member in place.

In further alternative embodiments, the second port housing 52 is in its entirety formed monolithically with the body 2.

However, it is preferred that the second port housing 52 comprises at least one separate element attached to the body 2, such as for instance a bushing 50a connected to a connection socket 50 or the outer surface of the body 2 or a distal element connected to a protrusion of the body 2. By using a separate element, the mounting of the second seal member 52 can be made particularly simple, especially if the second seal member 52 is to be inserted into the second port housing 51 from the outside. The interior of the second port housing 51 may then offer a form-fitting accommodation of the second seal member 52 without the necessity that the second seal member 52 is forced through a small opening.

In the second port housing 51, the second seal member 52 is accommodated. In the embodiment shown in Figures 1 and 2, the second seal member 52 comprises a distal wall in which the valve opening (see below) and a blind hole open at the proximal portion of the second seal member and terminating at the distal wall such that the interior of the blind hole is in communication with the valve opening. The blind hole as shown in Fig. 1 has a substantially constant circular cross-section. In alternative embodiments, the blind hole may have a different shape. The outer surface of the second seal member 52 shown in Fig. 1 has a taper approximately in its upper third. The taper can be of different degrees and extend over a different axial range. The taper can support the build-up of preload in the second seal member 52 (see below). However, the taper can also be completely absent.

The second seal member 52 comprises a valve opening 521 configured to provide a fluid-tight connection with a fluid manipulation device such as a syringe without a needle attached. The valve opening 521 is configured to be connected to the male connector of the fluid manipulation device such as a syringe. In the case of a typical syringe, the male connector is the tubular protrusion provided at the syringe's front surface. Preferably, the connection between the connector and the valve opening is fluid-tight.

In the embodiment shown in Fig. 1, the valve opening 521 is formed as a slit 522 in the elastic material of the distal wall of the second seal member 52. The slit 522 provides a channel through the distal wall. The connection of the fluid manipulation device and the second port 5 is made by inserting the male connector of the fluid manipulation device into the second port 5, wherein the male connector deforms the upper surface of the second seal member 52 in a way the slit opens and makes the flow of a fluid through the slit 522 and the male connector possible. In other embodiments, the slit is intended to be penetrated by the male connector.

If no fluid manipulation device is connected to the second port 5, the slit 522 is closed, i.e. the lateral surfaces (cut surfaces) of the slit touch each other. Preferably, the slit 522 is then closed in a fluid-tight manner. When the male connector of the fluid manipulation device is inserted, the slit 522 is expanded.

The valve opening 521 of the embodiment shown in Fig. 1 is formed by a single slit 522 in a cutting plane along the axial direction. In alternative embodiments, several slits may be provided whose cutting planes intersect each other along an axial line. In a section transverse to the axial direction, the cuts then form a cross or a star. Further, for instance a H-shaped section or other shapes of the valve opening may be possible.

The configuration and the size of the valve opening depends on how the connection part of the device with which a connection is intended is formed. If the connection with a conventional syringe is to be possible, the valve opening may, for example, be formed as a slit 522 as explained above.

Due to the elasticity of the material of the second seal member 52, a certain tolerance is given with regard to the dimensions of the male mating structure (male connector, i.e. the connection part) of the fluid manipulation device. This means that male mating structures of sizes that vary over a certain range can be inserted into a slit of a certain size. This is an advantage over rigid structures for making a needleless connection with a fluid manipulation device. For example, if the second port 5 were equipped with a rigid standardised female Luer structure and not with an elastic second seal member 52, essentially only connection with a standardised male Luer cone would be possible. The second port 5 designed according to the invention allows the connection with differently shaped and sized connection structures of the fluid manipulation device. If, for example, the connection to a syringe with a standard Luer cone is primarily intended, fluid manipulation devices with different connection structures can also be connected, e.g. a syringe with a smaller or larger cone.

Preferably, the valve opening 521 is resealable, i.e. after disconnecting the fluid manipulation device, the valve opening closes 521 such that the second elastic member 52 ensures that the second port 5 is closed in a fluid-tight manner again. More preferably, this resealing property is retained if a fluid manipulation device is connected and disconnected again several or even many times.

Preferably, the distal portion of the second seal member 52 is thin enough so that the distal portion of the second seal member 52 can be displaced vertically with relatively low axial force while the proximal portion bends or collapses, making the connection with a fluid manipulation device user-friendly. The bent or collapsed proximal portion then acts as a spring upon removal of the fluid manipulation device pushing the distal portion back to its initial position so that the second seal member 52 again tightly closes the second port 5, i.e. is resealing.

Preferably, the second seal member 52 is elastically preloaded. This may be achieved by selecting the shape and the size of the interior space of the second port housing 51 and the shape and the size of the second seal member 52 such that at least a portion of the second seal member 52 is elastically deformed when it is accommodated in the interior of the second port housing 51. This preload may provide or improve the connection between the second seal member 52 and the second port housing 51. Additionally or alternatively, this preload may provide or improve the self-sealing property of the second seal member 52 by facilitating the return of the second seal member 52 to its original shape and position during or after removal of the fluid manipulation device and by it thereby facilitating the fluid-tight closure of the valve opening 521.

In the embodiment shown in Fig. 1, the tapered portion of the second seal member 52 and the corresponding narrowed portion of the inner surface of the first port housing 51 provide an elastic deformation by which a preload force is exerted to the distal portion of the second seal member 52 in which the slit 522 is located, thus supporting self-sealing.

In addition or alternatively, in further preferred embodiments, the diameter of the second seal member 52 (measured transversely to the direction in which the fluid manipulation device is connected to the second port) is 18 mm or less, preferably 13.5 mm or less.

In addition or alternatively, in further preferred embodiments, the length of the second seal member 52 (measured in the direction in which the fluid manipulation device is connected to the second port) is between 10 and 15 mm and is longer than the cavity in which it is accommodated. This extra length creates an axial pre-load or contributes to an axial pre-load. Such axial pre-load provides a force that facilitates the return of the second seal member 52 to its original shape and position during or after removal of the fluid manipulation device and by it thereby facilitating the fluid-tight closure of the valve opening 521.

In the embodiment shown in Fig. 1, the second seal member 52 comprises a distal wall in which the valve opening is formed and a blind hole open at the proximal portion of the second seal member and terminating at the distal wall such that the interior of the blind hole is in communication with the valve opening. Preferably, the thickness of the distal wall is 1 mm or more and 6 mm or less, more preferably between 1 mm and 3 mm. Preferably, the diameter of the blind hole is 2 mm or more ensure proper fluid flow. More preferably, the diameter of the blind hole is 2.5 mm or more.

In specific embodiments the second seal member 52 comprises
- rubber, preferably vulcanized rubber, more preferably vulcanized polyisoprene rubber, and/or
- silicone, preferably silicone rubber, and/or
- at least one thermoplastic polymer, preferably at least one polymer selected from the group consisting of styrenic block copolymers, thermoplastic polyolefin-elastomers, thermoplastic vulcanizates, thermoplastic polyurethanes, thermoplastic copolyester, and thermoplastic polyamides.

Preferably, the distal surface of the second seal member 52 transitions essentially smoothly or entirely smoothly into the edge surface 512 of the second port housing 51.

In addition or alternatively, in further preferred embodiments, the second seal member 52 comprises an elastic material having a hardness between 25 and 55 shore A.

A peel-off foil 44 may be arranged atop of the first seal member 42. The peel-off foil 44 may be a foil comprising plastic and/or metallic materials such as an aluminium-plastic compound foil. The peel-off foil 44 may be connected to the cap 1 by welding onto the edge surface 412 of the first port housing 41.

The term "peel-off foil" indicates that preferably, the foil may be removed by the user by hand without the aid of any tools.

The peel-off foil 44 is removed before a piercing device is pierced through the first seal member 41. The peel-off foil 44 seals and protects the first sealing member 42, and in particular provides tamper evidence because the peel-off foil makes it easy to determine whether the first sealing member 42 is damaged or has already been punctured. Furthermore, the peel-off foil 44 provides additional tightness for the first port 4 and helps to ensure its mechanical integrity, for example, when the cap 1 is subjected to mechanical stress.

The second port 5 may have an equivalent peel-off foil 54.

The peel-off foils 44 and 54 arranged atop both seal members 42, 52 according to an embodiment of the invention are shown in Fig. 3. According to other embodiments, a peel-off foil is only arranged atop the first seal member 52 or atop the second seal member 52. According to other embodiments, none of the ports 4, 5 is provided with a peel-off foil.

It is preferred to design the distal surface of the second port housing 51 with a chamfered edge around the second opening 511. Such chamfered edge prevents material that is potentially molten and displaced when attaching the peel-off foil 54 that could contaminate the valve opening 522 and could be detrimental to a tight connection between the second port 5 and a fluid manipulation device.

Preferably, the body 2 comprises a polyolefin and more preferably consists thereof. In particular a material selected from the group consisting of polyethylene, polypropylene and ethylene-propylene copolymers or a mixture of multiple materials selected therefrom are used as polyolefin for the body 2.

According to the embodiment shown in Figures 1 and 2, the cap 1 further comprises a membrane 43. Preferably, the membrane 43 is formed monolithically with the body 2 of the cap 1. The membrane 43 is arranged underneath the first seal member 42 seen in a direction of insertion of a piercing device, wherein the membrane is adapted 43 to be pierced by the same piercing device that penetrates the first seal member. That is, when the piercing device is connected to the fluid container 100, it penetrates the first seal member 42 and, subsequently, the membrane.

In the embodiments shown in Figures 1 and 2, the proximal-distal direction for the first port 4 and the proximal-distal direction for the second port 5 are not parallel to each other but enclose an acute angle. In other words, the first port 4 and the second port 5 are in an angled arrangement.

The angled arrangement of the first port 4 and the second port 5 makes it possible or at least easier that a piercing device and a fluid manipulation device are simultaneously connected to the first port 4 and second port 5, respectively.

When the container 100 being provided with the cap 1 according to the invention is used for administering a liquid 300 to a patient, the container 100 and the cap 1 are usually oriented upside-down compared to the orientation shown in Fig. 4. Hence, the proximal-distal direction for the first port 4 is vertical whereas the proximal-distal direction for the second port 5 is inclined. The inclined orientation of the latter may make it easier for the user to administer a substance through the second port 5, e.g. by using a syringe.

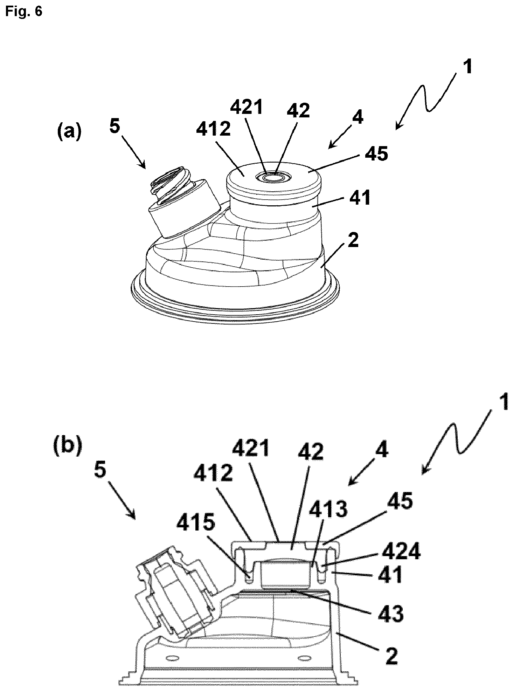

Figures 6 (a) and 6 (b) show a further preferred embodiment of the cap 1 according to the invention. According to this embodiment, the septum 42 is capped by a fastener 45. The fastener 45 may have a ring-like structure with a flat distal surface as shown in Figures 6 (a) and 6 (b). Other shapes such as a ring-like structure having a rounded distal surface are also possible.

The fastener 45 is connected to the distal portion of the first port housing 41. Together with a seat structure 413 within the first port housing 41, i.e. a step or the like at which the inner diameter of the first port housing 41 becomes narrower, the fastener provides a form-fit connection for the septum 42.

This configuration, in which the septum 42 is held by a fastener, is also referred to as "cassette design".

Optionally, the septum 42 may have a T-shaped lateral portion, i.e. a lateral portion provided with a protrusion 424 on its proximal side that corresponds to a recess 415 on the distal side of the seat structure 413. Preferably, the protrusion 424 and the recess 415 are ring-shaped. The protrusion 424 and the recess 415 reduce the tendency of the septum 42 to be pushed further into the first port housing 41 when pierced by a piercing device. That is, the protrusion 424 and the recess 415 further hold the septum 42 in place.

The fastener 45 may be made of the same material as the body 2.

For attaching the fastener 45 to the first port housing 45, for instance welding, in particular laser welding, gluing, or another suitable technique may be applied.

In addition to the form-fit connection provided by the fastener 45 and the seat structure 413, the septum 42 may be connected to the port housing 41 by another technique such as welding, in particular laser welding, or gluing. However, the cassette design is especially favoured if the septum cannot be attached to the port housing 41 by means of welding or gluing in an easy manner. For instance, it is difficult or even impossible to weld or glue a septum 42 made of vulcanised rubber to a first port housing 41 made of, e.g., polyolefin material. For a septum 42 made of such material, the cassette design is therefore preferred. Moreover, the cassette design may provide a stronger and therefore safer connection even if the septum may in principle be held in place by welding, gluing, etc. alone.

As the fastener 45 covers the distal surface or distal portion of the first port housing 41, the distal surface of the fastener 45 is considered as the distal edge surface 412 of the first port housing 41. In this sense, the fastener 45 may be seen as part of the first port housing 41. According to the embodiment shown in Figures 6 (a) and 6 (b), the distal surface 421 of the septum 42, i.e. the portion of the septum's distal side that is not covered by the fastener 45, is entirely flush with the distal edge surface 412. This configuration is achieved by a protrusion that fits into the opening of the fastener 45.

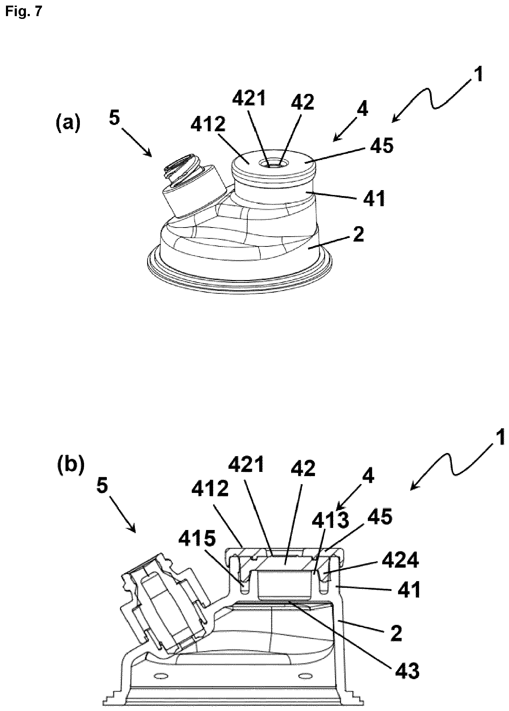

The septum 42 of the further embodiment shown in Figures 7 (a) and 7 (b) does not have such protrusion fitting into the fastener's opening but has a flat distal surface instead. Hence, the distal surface 421 of the septum 42 is only essentially flush with the edge surface 412. In the embodiment shown in Figures 7 (a) and 7 (b) the recess formed by the opening of the fastener 45 and the distal surface 421 of the septum 42 is only about 1.2 mm. With respect to the further structural aspects, the embodiment shown in Figures 7 (a) and 7 (b) corresponds to the embodiment shown in Figures 6 (a) and 6 (b).

## Claims

1. Cap (1) for a fluid container (100), in particular for a container (100) for a medical fluid (300), comprising:
(i) a body (2) comprising a container connection portion (3) adapted to be tightly connected to a container opening (201) of a container body (200),
(ii) a first port (4), comprising a first port housing (41), wherein the first port housing (41) comprises a first opening (411),
wherein the first port (4) further comprises an elastic first seal member (42) accommodated in the first port housing (41) and sealing the first opening (411),
wherein the first seal member (42) is formed as a pierceable septum (42),
wherein an outer surface (421) of the first seal member (42) is essentially flush or entirely flush with an edge surface (412) of the first opening (411), and
(iii) a second port (5) comprising a second port housing (51), wherein the second port housing (51) comprises a second opening (511),
wherein the second port (5) further comprises an elastic second seal member (52) accommodated in the second port housing (51) and sealing the second opening (511),
wherein the second seal member (52) comprises a valve opening (522) configured to provide a fluid-tight connection with a fluid manipulation device.

2. Cap (1) according to claim 1,
wherein the first port (4) is an administration port, wherein preferably the first seal member (42) is adapted to be pierced by a spike of an administration device selected from the group consisting of infusion set, transfusion set, and transfer device such that a fluid connection between the interior of the container and the administration device is provided, wherein more preferably the infusion set is an infusion set according to ISO 8536-4, the transfusion set is a transfusion set according to ISO 1135-4, and the transfer device is a transfer device according to ISO 22413, and/or
wherein the second port (5) is a needle-free port, wherein preferably the second port (5) is a medication port adapted to provide a fluid connection between the interior of the container and a fluid manipulation device, in particular a syringe, having a male connector, and/or wherein preferably the fluid connection between the interior of the container and the syringe or the other fluid manipulation device is a bidirectional fluid connection.

3. Cap (1) according to claim 1 or 2,
wherein the first seal member (42) and/or the second seal member (52) is overmoulded onto the body (2),
wherein preferably the first seal member (42) and the second seal member (52) are overmoulded onto the body (2), wherein more preferably the first seal member (42) and the second seal member (52) form a single elastic member overmoulded onto the body (2).

4. Cap (1) according to any of claims 1 to 3,
wherein the outer surface (421) of the first seal member (42) has a concave portion indicating the piercing location and preferably is essentially flat or entirely flat , and/or
wherein the first seal member (42) comprises rubber, preferably vulcanized rubber, and/or wherein the first seal member (42) comprises at least one thermoplastic elastomer, preferably at least one thermoplastic elastomer selected from the group consisting of styrenic block copolymers, thermoplastic polyolefin elastomers, thermoplastic vulcanizates, thermoplastic polyurethanes, thermoplastic copolyester, and thermoplastic polyamides, and/or
wherein the first seal member (42) comprises an elastic material having a hardness between 25 and 55 shore A, and/or
wherein the first seal member (42) comprises an elastic material including pigments and/or dyes capable of absorbing electromagnetic radiation, in particular electromagnetic radiation emitted by a welding laser,
wherein the second seal member (52) comprises rubber, preferably vulcanized rubber, more preferably vulcanized polyisoprene rubber, and/or wherein the second seal member (52) comprises silicone, preferably silicone rubber, and/or wherein the second seal member (52) comprises at least one thermoplastic polymer, preferably at least one polymer selected from the group consisting of styrenic block copolymers, thermoplastic polyolefin-elastomers, thermoplastic vulcanizates, thermoplastic polyurethanes, thermoplastic copolyester, and thermoplastic polyamides, and/or
wherein the second seal member (52) comprises an elastic material having a hardness between 25 and 55 shore A, and/or
wherein the body (2) comprises a polyolefin, preferably polypropylene and/or polyethylene, and/or
wherein the body (2) comprises a polymer material having a tensile modulus and/or flexural modulus of at least 700 MPa.

5. Cap (1) according to any of claims 1 to 4,
wherein the second seal member (52) is configured to provide a fluid-tight connection with the fluid manipulation device, wherein preferably the sealing valve opening (522) of the second seal member (52) is a self-sealing valve opening (522), wherein more preferably a self-sealing channel (521) for the male connector, in particular a self-sealing slit (521), is formed in the second seal member (52), wherein even more preferably the second seal member (52) is preloaded by an elastic deformation providing a spring force causing the channel (521) to close after a male connector inserted in the channel (521) is withdrawn from the channel (521).

6. Cap (1) according to any of claims 1 to 5,
wherein at least a portion of the first port housing (41) is formed monolithically with the body (2), wherein the portion formed monolithically with the body (2) accommodates the first seal member (42) and/or
wherein the second port housing (51) is formed as a separate member connected to the body (2), wherein preferably the second port housing (51) is permanently connected, in particular welded, to the body (2), and/or wherein preferably the second port housing (51) is connected to a connection socket (50) formed monolithically with the body (2) or permanently connected, in particular welded, to the body (2).

7. Cap (1) according to any of claims 1 to 6,
wherein the outer surface (521) of the second seal member (52) transitions essentially smoothly or entirely smoothly into the edge surface (512) of the second opening (511), and/or
wherein the second opening (511) has a chamfered edge surface (512), and/or
wherein the second port (5) is formed as female connector, and/or
wherein the second port housing (51) comprises a thread structure (53), preferably adapted for connecting with a male lock structure.

8. Cap (1) according to any of claims 1 to 7,
wherein the first port (4) and the second port (5) are arranged such that an administration device and a fluid manipulation device are simultaneously connectable to the first port (4) and second port (5), respectively,
and/or wherein the first port (4) and the second port (5) are in an angled arrangement,
wherein preferably the first port (4) and the second port (5) are arranged such that an angle between a direction of insertion of the administration device into the first port (4) and a direction of insertion of the fluid manipulation device into the second port (5) is between 15 and 45 degrees, in particular between 25 and 35 degrees, and/or
wherein the angle between a direction of insertion of the administration device into the first port (4) and a direction vertical to an opening formed by the container connection portion (3) is between 0 degrees and 30 degrees, preferably between 0 degrees and 10 degrees.

9. Cap (1) according to any of claims 1 to 8,
wherein a peel-off foil (44) is arranged atop the first seal member (42) seen in a direction of insertion of an administration device, in particular welded to the first port housing (41) and/or
wherein a peel-off foil (54) is arranged atop the second seal member (52) seen in a direction of insertion of a fluid manipulation device, in particular welded to the second port housing (51),
wherein preferably the peel-off foil (44, 54) arranged atop the first seal member (42) and/or atop the second seal member (52) seals the first opening (411) and/or the second opening (511), and/or
wherein preferably the peel-off foil (44, 54) arranged atop the first seal member (42) and/or atop the second seal member (52) provides tamper evidence, and/or
wherein preferably the peel-off foil (44, 54) arranged atop the first seal member (42) and/or atop the second seal member (52) provides tightness and integrity prior to use.

10. Cap according to any of claims 1 to 9,
wherein a membrane (43) is arranged underneath the first seal member (42) seen in a direction of insertion of an administration device, wherein the membrane (43) is adapted to be pierced by a piercing device of the administration device,
wherein preferably the membrane has a thickness between 0.1 mm and 0.5 mm, more preferably between 0.15 and 0.25 mm.

11. Fluid container (100), in particular fluid container (100) for a medical fluid (300), comprising
- a hollow container body (200) having a container body opening portion (201)
- a cap (1) according to any of claims 1 to 10, wherein the body (2) of the cap (1) is in fluid-tight connection with the opening portion (201).

12. Fluid container according to claim 11, wherein the container body (200) is defined by anyone of the following structural features (i) to (v), respectively alone or in combination:
(i) the container body (200) is at least partly filled with a fluid (300), in particular a medical fluid (300),
(ii) the container body (200) is collapsible,
(iii) the container body (200) is a semi-rigid container body (200),preferably is a collapsible flexible bag, more preferably made of multilayer film,
(iv) the container body (200) comprises a polyolefin material, preferably at least a wall of the container body (200) is made of polypropylene and/or polyethylene,
(v) the container body (200) is manufactured using a blowing technique, preferably a technique selected from extrusion blow moulding, injection stretch blow moulding, or blow-fill-seal technology, more preferably extrusion blow moulding.

13. Method of manufacturing a cap (1) of any of claims 1 to 10,
comprising the steps
A) manufacturing or providing the body (2),
B) manufacturing or providing the first seal member (42),
C) manufacturing or providing the second seal member (52),
D) mounting the first seal member (42) into the body (2), wherein the outer surface (421) of the first seal member (42) is essentially flush or entirely flush with the edge surface (412) of the first opening (411),
E) mounting the second seal member (52),
F) optionally arranging a peel-off foil (44, 54) atop the first seal member (42) and/or atop the second seal member (52),
wherein preferably mounting a seal member (42, 52) includes inserting it into the respective first or second port housing (41, 51) and fixing it in place, wherein the first seal member (42) is more preferably fixed in place by welding, in particular laser welding, and/or wherein more preferably the second seal member 52 is fixed in place by a form-fit connection with the second port housing (52).

14. Method according to claim 13,
wherein the first seal member (42) is manufactured and mounted by overmoulding it into the first port housing (41) and/or
wherein the second seal member (52) is manufactured and mounted by overmoulding it into the second port housing (51).

15. Method according to any of claims 13 to 14,
wherein, for mounting the first seal member (42), the first seal member (42) is inserted into the first port housing (41) in a direction corresponding to a direction in which an administration device is inserted into the first port (4),
and/or
wherein, for mounting the second seal member (52), the second seal member (52) is brought into position in a direction corresponding to a direction in which a fluid manipulation device is inserted into the second port (5), wherein preferably the second port housing (51) is mounted atop the second seal member (52).
